# EUROPÄISCHE PATENTSCHRIFT

(11) **EP 2 802 286 B1**
(45) Veröffentlichungstag und Bekanntmachung des Hinweises auf die Patenterteilung: **04.01.2017**
(21) Anmeldenummer: 13700635.9
(22) Anmeldetag: 09.01.2013
(51) Int. Cl.: A61C 17/08, A61B 1/247

(54) **SPIEGELSAUGER**
MIRRORED SALIVA EJECTOR
POMPE À SALIVE À MIROIR

(30) Priorität: 09.01.2012 DE 102012100119
(43) Veröffentlichungstag der Anmeldung: 19.11.2014
(73) Patentinhaber: Clasen, Stephan, 48149 Münster (DE); Kayser, Martin, 50968 Köln (DE)
(72) Erfinder: Clasen, Stephan, 48149 Münster (DE); Kayser, Martin, 50968 Köln (DE)
(74) Vertreter: Patentanwälte Bauer Vorberg Kayser
(86) Internationale Anmeldenummer: PCT/EP2013/050263
(87) Internationale Veröffentlichungsnummer: WO 2013/104647

(56) Entgegenhaltungen:
- EP-A1- 2 181 643
- DE-A1-102005 000 929
- SE-C2- 528 627

## Beschreibung

Die vorliegende Erfindung betrifft einen Spiegelsauger zum Absaugen von Flüssigkeiten und Partikeln aus einem Mundraum eines Patienten, mit einem hohlen Grundkörper, der eine Außenfläche, eine Innenfläche, eine Längsachse und eine Ansaugöffnung aufweist, wobei die Innenfläche einen durch die Ansaugöffnung einsehbaren Spiegel aufweist und der Grundkörper aus einem ersten Grundkörperteil und einem zweiten Grundkörperteil gebildet ist, die über Verbindungsflächen fest miteinander verbunden sind. Weiterhin betrifft die Erfindung ein Verfahren zur Herstellung eines solchen Spiegelsaugers.

Bei zahnmedizinischen Behandlungen ist es oftmals notwendig, anfallende Flüssigkeiten oder gelöste Partikel vom Behandlungsbereich abzuführen. Beispielsweise ist es bei einer zahnmedizinischen Behandlung notwendig, Speichel, Spraywasser und Blut während der Behandlung abzusaugen. Auch kann Wasser, beispielsweise zum Reinigen oder nach Anwenden einer Multifunktionsspritze anfallen, das abgesaugt werden muss. Hierzu werden üblicherweise Absauger verwendet, die in der Regel aus einem röhrenförmigen Körper aus Kunststoff gebildet sind, an dessen Ende ein Schlauch befestigt ist, der wiederum mit einer Pumpe verbunden ist. Durch den Schlauch bzw. den Absauger werden dann die störenden Flüssigkeiten und Festkörper abgesaugt.

Auch in anderen medizinischen Bereichen, insbesondere bei chirurgischen Behandlungen ist es häufig notwendig, Blut, Wasser und/oder Knochenpartikel und ähnliches während der Operation abzusaugen.

Üblicherweise wird der Absauger nicht vom behandelnden Arzt bzw. behandelnden Chirurgen selbst, sondern von einer oder einem Assistenten geführt und gehalten. Dies ist deshalb notwendig, weil der behandelnde Arzt meist beide Hände für seine Behandlung benötigt.

Nachteilig bei der beschrieben Vorgehensweise ist, dass zwei Personen, nämlich der behandelnde Arzt und die assistierende Person sehr eng beieinander an der zu behandelnden Person um den zu behandelnden Bereich stehen oder sitzen müssen. Dies kann gerade dann, wenn es sich um verhältnismäßig schwierige oder feinmotorisch anspruchsvolle Eingriffe handelt, vom behandelnden Arzt und/oder Patienten als störend empfunden werden. Hinzu kommt, dass sich zumindest zwischenzeitlich drei Werkzeuge im Mund des Patienten befinden.

Ein weiterer Nachteil ergibt sich daraus, dass der behandelnde Arzt, dann wenn er beispielsweise ein medizinisches Werkzeug, einen Absauger und einen Spiegel gebrauchen muss, auf eine assistierende Person angewiesen ist. Dies ist gerade bei Zahnärzten ein besonderer Nachteil, weil die zahnärztlichen Behandlungen oftmals problemlos ohne eine assistierende Person durchgeführt werden könnten, dies jedoch daran scheitert, dass Flüssigkeiten oder Partikel abgesaugt werden müssen.

Aus der DE 102005000929 A1 ist ein medizinischer Absauger bekannt, bei dem die Innenfläche eine durch die Ansaugöffnung sichtbare verspiegelte Oberfläche aufweist. Die erfindungsgemäße spiegelnde Beschichtung ermöglicht es dem Benutzer, den medizinischen Absauger sowohl als Absauger zum Abführen von Flüssigkeiten und Partikeln, als auch gleichzeitig als Spiegel zu benutzen. Mit Hilfe dieses Absaugers ist es ihm möglich, Behandlungen ohne eine assistierende Person durchzuführen für die er ansonsten Hilfe benötigen würde. Da der permanente Luftstrom ein Beschlagen des Spiegels verhindert, ist beispielsweise auch ein Einsatz bei Trockenarbeiten möglich.

Die Grundidee der Kombination der beiden Geräte ist grundsätzlich gut, allerdings hat sich die praktische Umsetzung der Idee bzw. die Herstellung eines solchen Absaugers als äußerst schwierig erwiesen. Die Verspiegelung der Innenfläche des Grundkörpers beispielsweise durch Auftragen einer spiegelnden Beschichtung hat sich als untauglich erwiesen, weil die Qualität der spiegelnden Beschichtung nicht ausreichend gut ist. Das Spiegelbild ist unscharf, verzerrt oder unklar. Auch ist es nicht gelungen, anstelle der Verspiegelung des Grundkörpers selbst einen separaten Spiegel dauerhaft und unverlierbar mit dem Grundkörper zu verbinden.

Die Aufgabe der vorliegenden Erfindung besteht darin, einen Spiegelsauger bereitzustellen, der eine ausreichend hochwertige Spiegelfläche im Bereich der Ansaugöffnung aufweist. Insbesondere soll die Spiegelfläche fest und unverlierbar im Spiegelsauger bzw. in dessen Grundkörper gehalten sein. Der Spiegelsauger soll dabei kostengünstig herstellbar und einfach einzusetzen sein. Weiterhin ist es Aufgabe der Erfindung, ein Verfahren zur Herstellung eines Spiegelsaugers mit den genannten Vorteilen vorzuschlagen.

Erfindungsgemäß wird die Aufgabe durch einen Spiegelsauger mit den Merkmalen des Patentanspruchs 1 gelöst. Ein erfindungsgemäßes Herstellungsverfahren ist in Patentanspruch 11 angegeben.

Demnach weist der erfindungsgemäße Spiegelsauger zwei Grundkörperteile mit jeweils einer Ausnehmung auf, die im zusammengesetzten Zustand der beiden Grundköperteile eine um den gesamten Außenumfang des Spiegels umlaufende Spiegelhalterungsnut ausbilden, wodurch der Spiegel zwischen den beiden Grundköperteilen unverlierbar gehalten ist.

Die Herstellung Spiegelsaugers erfolgt durch ein Verfahren mit den folgenden Herstellungsschritten:
- Fertigen eines ersten Grundkörperteils und eines zweiten Grundkörperteils, und dabei
   - Ausbilden mindestens einer Verbindungsfläche je Grundkörperteil,
   - Ausbilden einer Spiegelhalterungsnut je Grundkörperteil, die derart angeordnet und ausgeformt sind, dass der Spiegel im zusammengesetzten Zustand der beiden Grundköperteile in dieser gehalten ist,
- Einsetzen des Spiegels in die Spiegelhalterungsnut und Zusammenfügen der beiden Grundkörperteile an den Verbindungsflächen,
- Befestigen der Grundkörperteile aneinander im Bereich der Verbindungsflächen.

Die Formulierung, dass der Spiegel unverlierbar gehalten ist, meint, dass dieser bestimmungsgemäß nicht aus dem Grundkörper entfernbar ist bzw. ein Entfernen des Spiegels nur durch unsachgemäße Veränderung oder Zerstörung des Grundkörpers möglich ist.

Erfindungsgemäß bleibt der Spiegel über die Lebensdauer des Spiegelsaugers also fest mit dem Grundkörper verbunden. Vorteilhafterweise bestehen der Grundkörper und der Spiegel aus Materialien, welche ein mehrmaliges Benutzen des Spiegelsaugers ermöglichen. Der Grundkörper und der Spiegel werden zwischen zwei Behandlungen gemeinsam desinfiziert bzw. sterilisiert, aufgrund der festen Verbindung der beiden Teile ist es nicht notwendig, diese vor der Reinigung bzw. Desinfektion voneinander zu trennen. Hierdurch wird der mit der Reinigung und Sterilisation verbundene Arbeitsablauf in einer Arztpraxis vereinfacht und beschleunigt.

Durch die erfindungsgemäße Spiegelhalterungsnut ist der Spiegel sicher und dauerhaft gehalten. Die Spiegelhalterungsnut verläuft vorteilhafterweise über den gesamten Außenumfang des in der Regel kreisförmigen oder ovalen Spiegels. Alternativ ist es aber auch möglich, dass die Spiegelhalterungsnut nur Bereiche des Außenumfangs des Spiegels umgreift. Durch die Spiegelhalterungsnut ist der Spiegel im Querschnitt vorzugsweise nicht nur seitlich, sondern auch von oben gehalten.

Es hat sich gezeigt, dass die Fertigung des Spiegelsaugers dann besonders einfach ist, wenn der Grundkörper aus zwei Grundkörperteilen gebildet ist, die im Wesentlichen in Längsrichtung entlang einer Längsachse des Grundkörpers bzw. des Spiegelsaugers verlaufen. Beide Grundkörperteile weisen vorzugsweise etwa eine Hälfte der Spiegelhalterungsnut auf. Ist der Spiegel beispielsweise kreisförmig, weist jedes Grundkörperteilen vorzugsweise eine Spiegelhalterungsnut auf, die im zusammengesetzten Zustand des Spiegelsaugers etwa 180° des Spiegels umfasst.

Der Verlauf der Verbindungsflächen der beiden Grundkörperteile kann grundsätzlich aber auch quer zur Längserstreckung des Spiegelsaugers verlaufen.

In einer besonders vorteilhaften Ausführungsvariante ist der Spiegel im vertikalen Querschnitt derart ausgeführt, dass der Durchmesser des Spiegels ausgehend von seiner Spiegelfläche zunimmt, also im eingesetzten Zustand in Richtung der Grundkörperunterseite. Der Spiegel kann beispielsweise im Querschnitt trapezförmig ausgeführt sein.

Die Spiegelhalterungsnut, die den Spiegel umlaufend umgibt, weist einen dazu korrespondierenden vertikalen Querschnitt auf, ihre sich parallel zur Spiegelfläche erstreckende Tiefe nimmt in Richtung der Grundkörperunterseite ebenfalls zu. Durch diese Ausführungsform ist gewährleistet, dass der Spiegel im zusammengesetzten Zustand des Spiegelsaugers von der Spiegelhalterungsnut umschlossen und gehalten ist. Sind die beiden Grundkörperteile miteinander verbunden, ist ein Entfernen des Spiegels ohne Zerstörung des Grundkörpers nicht mehr möglich.

Es hat sich gezeigt, dass ein sich möglicherweise ergebender Raum seitlich in der Spiegelhalterungsnut und unterhalb des Spiegels dann besonders dicht verschlossen werden kann, wenn die Spiegelhalterungsnut die Spiegelfläche von oben oder eine Spiegelseitenfläche vollumfänglich kontaktiert. Hierzu steht die Spiegelhalterungsnut zumindest bereichsweise unter Vorspannung, die beim Einsetzen des Spiegels überwunden werden muss. Der Spiegel drückt im zusammengesetzten Zustand der beiden Grundkörperteile gegen den entsprechenden Bereich der Spiegelhalterungsnut, so dass dieser Bereich aufgrund der Elastizität des Materials und/oder der Form in Kontakt mit dem Spiegel bleibt. Die sich so ergebende verbesserte Abdichtung ist hinsichtlich der Sterilisation des Spiegelabsaugers besonders vorteilhaft, da sich in dem freien Raum zumindest weniger oder sogar keine Keime ansammeln können.

Bei der erfindungsgemäßen Querschnittsform des Spiegels bei der dessen Durchmesser ausgehend von der Spiegelfläche zunimmt, weist die Spiegelhalterungsnut eine entsprechende Haltewandung auf, die im vertikalen Querschnitt der Spiegelhalterungsnut seitlich bzw. von schräg oben gegen die Spiegelseitenfläche drückt. Die Haltewandung wirkt als Dichtlippe und legt sich ähnlich wie ein Simmering umlaufend an die Spiegelseitenfläche an.

Die trapezförmige Ausführungsvariante des Spiegels mit der entsprechenden Ausführung der Spiegelhalterungsnut hat weiterhin den Vorteil, dass der Spiegel in einen Grundkörperboden eingelassen werden kann, so dass die Spiegelfläche in etwa mit einem inneren Grundkörperboden bündig abschließt. Dies hat zum einen Vorteile hinsichtlich der Raumausnutzung, wesentlich ist jedoch auch, dass das Absaugverhalten des Spiegelsaugers dadurch deutlich verbessert ist. Flüssigkeit und Partikeln können über den insgesamt glatten und in etwa eine durchgehende Höhe aufweisende Grundkörperboden widerstandslos abgesaugt werden. Hinzu kommt, dass die Geräuschentwicklung beim Absaugen aufgrund der glatten Flächen und dadurch verminderten Luftverwirbelungen deutlich geringer ist. Die Geräuschentwicklung ist ein wesentliches Kriterium für die Qualität von Absaugern, da die Geräusche unmittelbar am oder im Kopf des Patienten entstehen und erheblich stören können. Weiterhin ist es für einen Zahnarzt, der täglich mehrere Stunden mit einem Absauger arbeitet, wichtig, dass dieser geringe oder zumindest einigermaßen angenehme Geräusche verursacht. Ein Überstand des Spiegels gegenüber dem Grundkörperboden von bis zu 0,3 mm wird im Sinne der Erfindung noch als bündig angesehen.

Die Ansaugöffnung des rohrförmigen Grundkörpers verläuft nicht rechtwinklig zur Längsachse, sondern ist schräg zu ihr ausgeführt. Dadurch ergibt sich eine schräg zulaufende spitze Form des Absaugers, wodurch dieser leichter beispielsweise zwischen Wange und Zähne einführbar ist. Der auf oder im Grundkörperboden angeordnete Spiegel, ist durch die Ansaugöffnung einsehbar. Das bedeutet, dass der Absauger genau so, wie es der behandelnde Zahnarzt gewohnt ist, an den Zahn herangeführt wird, nämlich mit der Ansaugöffnung in Richtung des Zahns, wodurch der Zahnarzt dann durch die Ansaugöffnung über die spiegelnde Innenfläche auf den Zahn bzw. behandelnden Bereich blicken kann. Der Spiegel befindet sich nicht vor der Ansaugöffnung, sondern in Strömungsrichtung der angesaugten Luft hinter der Ansaugöffnung, also innerhalb des Grundkörpers. Hierdurch wird erreicht, dass der Spiegelsauger nicht durch einen vorgelagerten Spiegel verlängert ist, was die Ansaugleistung vermindern würde. Die Integration des Spiegels in den Grundkörper hinein führt dazu, dass die Ansaugöffnung nahe an die abzusaugende Flüssigkeit oder die abzusaugenden Partikel herangeführt werden kann, ohne dass dies durch einen vorgelagerter Spiegel dies behindert wird.

Erfindungsgemäß kann zusätzlich zum innerhalb des Grundkörpers angeordneten Spiegel als weitere Spiegelfläche die Außenfläche des Spiegelsaugers, zumindest bereichsweise mit einer spiegelnden Oberfläche versehen sein.

In einer weiteren Ausführungsform der Erfindung ist der Absauger in seinem die Ansaugöffnung aufweisenden Endbereich aufgeweitet ausgeführt, um dadurch insbesondere die innen liegende Spiegelfläche zu erhöhen. Je nach gewünschter Funktion kann es sinnvoll sein, wenn die spiegelnde Beschichtung eine vergrößernde oder verkleinernde Wirkung hat.

In einer besonders vorteilhaften Ausführungsvariante ist der Spiegel ausschließlich und ohne zusätzlichen Klebstoff in der Spiegelhalterungsnut gehalten. Die klebstofffreie Befestigung ist zum einen kostengünstiger, zum anderen fallen somit sämtliche durch den Klebstoff begründete Schwierigkeiten und Nachteile weg. Der Klebstoff muss zum Beispiel für die Verwendung des Spiegelsaugers im Mund des Patienten entsprechende hygienische und/oder medizintechnische Zulassungen aufweisen. Er muss für den Patienten unbedenklich sein.

In einer weiteren Ausführungsvariante kann aber auch die Verwendung eines unbedenklichen Klebstoffes zur zusätzlichen Befestigung des Spiegels vorgesehen sein.

Der erfindungsgemäße Spiegelsauger kann aus jedem geeigneten Material hergestellt sein, es bietet sich jedoch aus Kostengründen eine Fertigung aus einem Kunststoffmaterial an. Erfindungsgemäß hat sich neben der Verwendung von beispielsweise Polypropylen insbesondere auch die Fertigung der Grundkörpers aus Polyester als vorteilhaft erwiesen. Polyester weist hinsichtlich der Oberflächenbeschaffenheit des fertigen Produkts gegenüber anderen Kunststoffen deutliche Vorteile beispielsweise in Bezug auf den Glanzgrad, die Kratzfestigkeit sowie die Oberflächenglätte auf.

Vorteilhafterweise können die beiden Grundkörperteile über eine Nut/Feder-Verbindung miteinander verbunden sein. Die Verbindungsflächen weisen entsprechend jeweils eine Nut oder eine auf Feder auf. Bei der Herstellung des Spiegelsaugers wird die Feder in die Nut gedrückt und die beiden Grundkörperteile werden miteinander verklebt oder vorzugsweise miteinander verschweißt. Die Nut/Feder-Verbindung hat den wesentlichen Vorteil, dass die beiden Grundkörperteile in ihrer Position während des Verklebens des oder Verschweißens relativ zueinander ausgerichtet bleiben und nicht gegeneinander verschoben werden können. Dies ist gerade bei der Nutzung der Spiegelhalterungsnut ein entscheidender Vorteil, da ein ungewollter Versatz der beiden Grundkörperteile und damit der beiden Hälften oder Bereiche der Spiegelhalterungsnut eine sichere und gegebenenfalls flüssigkeitsdichte Befestigung des Spiegels erschwert oder verhindert. Insbesondere dann, wenn der Grundkörper entlang seiner Längsachse in zwei Grundkörperteile unterteilt ist, also die Verbindungsflächen im Wesentlichen in Längsrichtung des Grundkörpers verlaufen, macht sich schon ein minimaler Versatz der beiden Grundkörperteile in Längsrichtung negativ bemerkbar. Ein Versatz der beiden Grundkörperteile zueinander führt zwangsläufig einer vorstehenden Kante, die gerade an der Vorderseite des Spiegelsaugers im Bereich des Spiegels unakzeptabel ist, da diese den Patienten stören würde.

Die Nut/Feder-Verbindung kann sich in Form von einzelnen Nut/Feder-Verbindungsabschnitten über mehrere Bereiche der Verbindungsflächen erstrecken, sie kann aber auch als nur eine durchgehende Nut/Feder-Verbindung, vorzugsweise entlang der gesamten Länge der Verbindungsflächen verlaufen. Ist der Grundkörper entlang seiner Längsachse in zwei Grundkörperteile geteilt, ist es von besonderem Vorteil, wenn die Nut/Feder-Verbindung im Längsschnitt zumindest unterhalb des Spiegels und über dessen gesamte Breite verläuft. Hierdurch wird sichergestellt, dass durch die exakte Fixierung der beiden Grundkörperteile aneinander die Befestigung des Spiegels in der Spiegelhalterungsnut den gewünschten Anforderungen entspricht.

Eine besonders hochwertige Verbindung der beiden Grundkörperteile ergibt sich dann, wenn sich die Nut/Feder-Verbindung im Längsschnitt unterhalb des Spiegels über seine gesamte Breite und darüber hinaus zwischen einer Grundkörpervorderkante und einer Spiegelvorderkante erstreckt. Die Nut-Federverbindung weist somit einen in etwa horizontal und im Bereich vor der Spiegelvorderkante einen in etwa vertikal verlaufenden Bereich auf. Die beiden Grundkörperteile sind über eine solche Nut/Feder-Verbindung sowohl in vertikaler als auch in horizontaler Richtung gehalten.

Erfindungsgemäß ist bei der Fertigung des Spiegelsaugers vorgesehen, dass nach einem Zusammendrücken der Nut-Feder-Verbindung ein minimaler Spalt zwischen den beiden Grundkörperteilen verbleibt. In diesem Spalt kann in einem nächsten Fertigungsschritt, dem Verkleben oder Verschweißen der beiden Grundkörperteile miteinander, Klebstoff oder durch den Schweißprozess aufgeweichtes Material des Grundkörpers aufgenommen werden. Dies führt letztendlich zu einer ausgesprochen glatten äußeren Oberfläche des Spiegelsaugers.

Obwohl ein Verkleben der Grundkörperteile miteinander grundsätzlich möglich ist, hat es sich als besonders vorteilhaft erwiesen, die beiden Grundkörperteile miteinander zu verschweißen und keinen Klebstoff zu verwenden. Die mit dem Klebstoff verbundenen Nachteile können dadurch vermieden werden. Durch die Ausbildung und Dimensionierung des verbleibenden Spalts nach dem Zusammenrücken der Nut/Feder-Verbindung kann gerade bei einem Verschweißen der beiden Grundkörperteile eine geschlossen und glatte Oberfläche ausgebildet werden.

Der erfindungsgemäße Spiegelsauger weist in einer besonders vorteilhaften Ausführungsvariante sowohl die erfindungsgemäße Spiegelhalterungsnut mit den beschriebenen vorteilhaften Ausführungsvarianten sowie die ebenfalls bereits beschriebene erfindungsgemäße Nut/Feder-Verbindung auf. Alternativ kann es aus Kostengründen oder aufgrund anderer Anforderungen und Gründe auch möglich sein, dass der Spiegelsauger nur entweder die vorteilhafte Spiegelhalterungsnut oder die vorteilhafte Nut/Feder-Verbindung aufweist.

Erfindungsgemäß kann vorgesehen sein, dass bei der Fertigung des Spiegelsaugers kein Klebstoff verwendet wird, weder für die Befestigung des Spiegels, noch für die Verbindung der Grundkörperteile miteinander.

Der Grundkörper kann vorteilhafterweise in seinem Verlauf zusätzlich zur Ansaugöffnung zumindest eine Zusatzöffnung aufweisen. Dies hat den Vorteil, dass dann, wenn die Ansaugöffnung beispielsweise durch die Wange oder Zunge des Patienten verschlossen wird, Luft durch die Zusatzöffnung angesaugt kann. Dies verhindert den für den Patienten unangenehmen Unterdruck und das damit verbundene Ansaugen der Wange oder Zunge an die Ansaugöffnung. Wesentlich ist auch, dass durch den Bypass der Zusatzöffnungen ein Rückströmen von Flüssigkeiten in Richtung der Ansaugöffnung, also zum Patienten, verhindert wird. Weiterhin ist im Bereich der Zusatzöffnung in Strömungsrichtung der Luft hinter der Öffnung auf der äußeren Oberfläche des Grundkörpers eine Erhöhung vorgesehen. Diese Erhöhung verhindert, dass durch die angesaugte Luft ein hochfrequenter Ton entsteht. Töne mit hoher Frequenz sind für den Patienten und den behandelnden Arzt unangenehm, weswegen der erfindungsgemäße Spiegelsauger auch diesbezüglich gegenüber bekannten Absaugern Vorteile aufweist.

Vorteilhafterweise kann der Spiegelsauger im Bereich seiner Ansaugöffnung eine Lichtquelle aufweisen, die den Bereich beleuchtet, der über den Spiegel sichtbar sein soll. Dies kann beispielsweise durch einen Lichtleiter gewährleistet sein, der sich entlang des Grundkörpers erstreckt. Die Lichtquelle kann direkt im Bereich der Anschlussöffnung oder sogar extern angeordnet sein, wenn sie in den Lichtleiter einstrahlt. Die Kombination von Spiegel und Lichtquelle erlaubt eine optimale Einsicht in den zu behandelnden Bereich.

Grundsätzlich ist es möglich, den erfindungsgemäßen Spiegelsauger in unterschiedlichen Dimensionen herzustellen. Der Spiegelsauger kann zum Beispiel unterschiedliche Durchmesser aufweisen, insbesondere auch im Bereich der Anschlussöffnung, an die der Schlauch zum Ableiten der Flüssigkeiten und Partikel angebracht wird. Auch sind größere und kleinere Spiegeldurchmesser für verschiedene Anwendungsbereiche sinnvoll.

In den nachfolgenden Figuren wird die Erfindung näher erläutert. Die gezeigten Ausführungsbeispiele sollen dabei die Erfindung nicht einschränken, sondern diese lediglich verdeutlichen.

Es zeigen:
- Fig. 1:: einen erfindungsgemäßen Spiegelsauger in perspektivischer Darstellung von schräg oben,
- Fig. 2:: den erfindungsgemäßen Spiegelsauger von oben,
- Fig. 3:: den erfindungsgemäßen Spiegelsauger im Längsschnitt,
- Fig. 4:: eine vergrößerte Darstellung des Bereichs A aus Figur 1,
- Fig. 5:: den erfindungsgemäßen Spiegelsauger im Vertikalschnitt B-B,
- Fig. 6:: eine vergrößerte Darstellung des vorderen Bereichs des Spiegelsaugers im Längsschnitt,
- Fig. 7:: eine perspektivische Darstellung des vorderen Bereichs des Spiegelsaugers von schräg vorne.

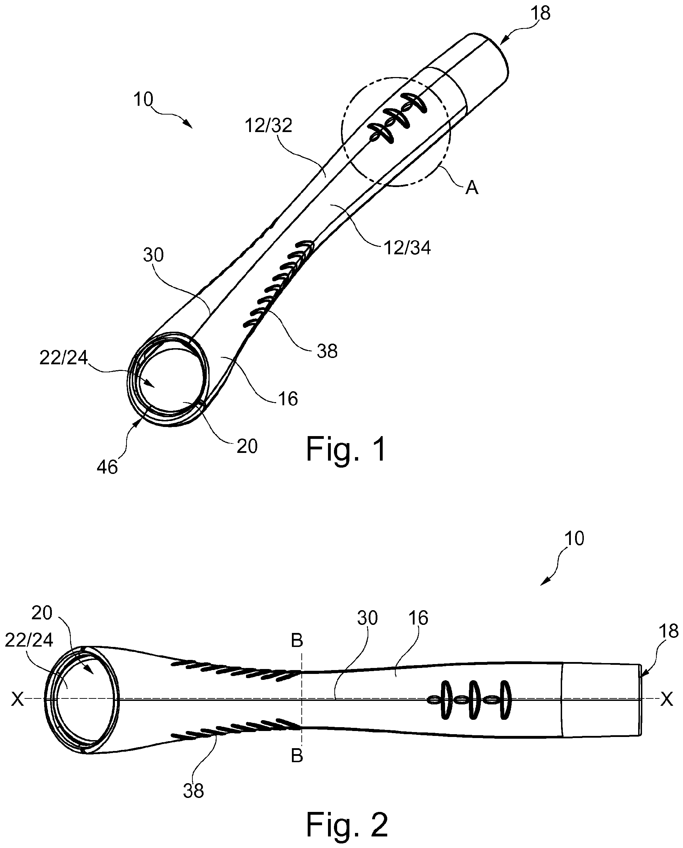

Wie sich aus den Figuren ergibt, weist ein erfindungsgemäßer Spiegelsauger 10 einen hohlen rohrförmigen Grundkörper 12 mit einer Innenfläche und einer Außenfläche 16 auf. Weiterhin weist der Grundkörper 12 eine Längsachse X-X auf (vergl. Fig. 2). Die insbesondere in Figur 1 erkennbare Bogenform des Spiegelsaugers 10 hat den Vorteil, dass dieser leichter an die zu behandelnde Stelle herangeführt werden kann.

Der Grundkörper 12 weist eine Anschlussöffnung 18 und eine Ansaugöffnung 20 auf. Durch die Ansaugöffnung 20 werden die abzusaugenden Flüssigkeiten oder Partikel eingesogen und durch die Anschlussöffnung 18 über einen nicht gezeigten Schlauch abgeleitet.

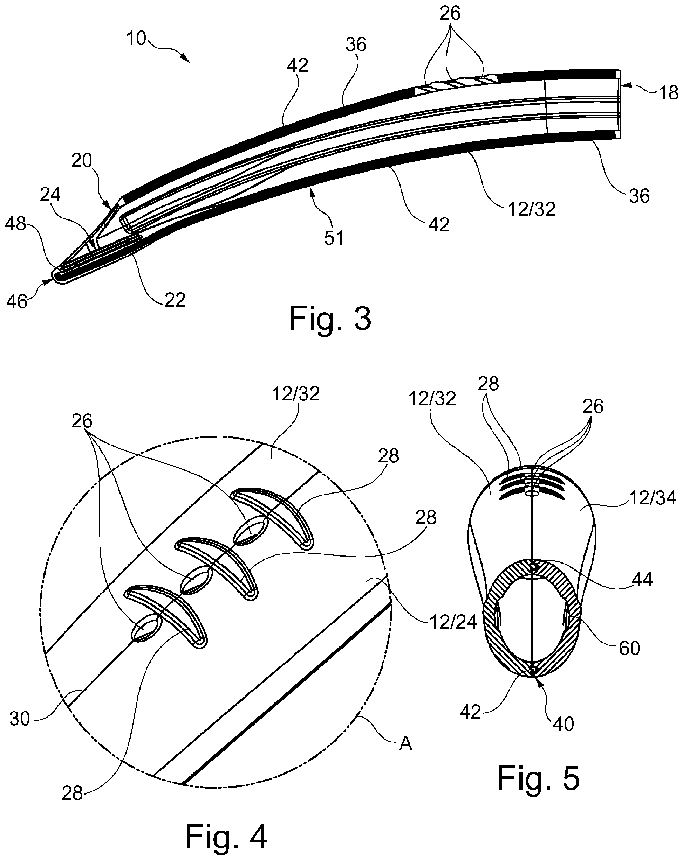

Erfindungsgemäß ist innerhalb des Grundkörpers 12 ein Spiegel 22 im Bereich der Ansaugöffnung 20 angeordnet, der durch die Ansaugöffnung 20 einsehbar ist. Entsprechend ist dessen Spiegelfläche 24 der Ansaugöffnung 20 zugewandt. Insbesondere die Figuren 3, 6 und 7 verdeutlichen, dass der Spiegel 22 vollständig innerhalb des Grundkörpers 12, also in Strömungsrichtung der abzusaugenden in Luft hinter der Ansaugöffnung 20 angeordnet ist. Die angesaugte Luft wird über die Spiegelfläche 24 geleitet, wodurch ein Beschlagen der Spiegelfläche 24 effektiv verhindert wird.

Der Spiegelsauger 10 weist Zusatzöffnungen 26 auf, durch die ebenfalls Luft angesaugten wird. Die Zusatzöffnungen 26 verhindern einen Unterdruck innerhalb des Grundkörpers 12, wenn die Ansaugöffnung 20 beispielsweise durch die Zunge oder Wange des Patienten verschlossen wird. Im gezeigten Ausführungsbeispiel sind drei Zusatzöffnungen 26 vorgesehen, denkbar ist aber auch nur eine einzige Zusatzöffnung 26 oder auch mehr als drei Zusatzöffnungen 26. Wie Figur4verdeutlicht ist in Strömungsrichtung der angesaugten Luft hinter jeder Zusatzöffnung 26 auf der Außenseite 16 des Grundkörpers 12 jeweils eine Erhöhung 28 vorgesehen, die die Frequenz eines durch die angesaugte Luft entstehenden Tons positiv beeinflusst.

In Figur 1 und Figur 2 ist weiterhin eine Trennlinie 30 erkennbar, die sich daraus ergibt, dass der Grundkörper 12 aus einem ersten Grundkörperteil 32 und einem zweiten Grundkörperteil 34 gebildet ist. Die beiden Grundkörperteile 32, 34 sind in etwa entlang der Längsachse X-X über Verbindungsflächen 36 des Grundkörpers 12 zusammengesetzt.

Auf der Außenfläche 16 des Grundkörpers 12 sind weiterhin Profilelemente 38 erkennbar, die einen sicheren Griff des Spiegelsaugers 10 und ein Abrutschen der Finger des behandelnden Arztes verhindern.

Figur 3 zeigt das erste Grundkörperteil 32 und die entsprechenden Verbindungsflächen 36. Weiterhin wird die Position des Spiegels 22 deutlich, der im Längsschnitt im vorderen Bereich des Spiegelsaugers 10 unterhalb der Ansaugöffnung 18 positioniert ist.

Erfindungsgemäß sind die beiden Grundkörperteile 32,34 über eine Nut/Feder-Verbindung 40, die in den Verbindungsflächen 36 angeordnet ist, miteinander verbunden. Die Nut/Feder-Verbindung 40 ist insbesondere in Figur 5 gut erkennbar. Das erste Grundkörperteil 32 weist eine Feder 42 und das zweite Grundkörperteil 34 eine entsprechende Nut 44 auf. Bei der Fertigung des Spiegelsaugers 10 werden die Federn 42 in die korrespondierenden Nuten 44 eingefügt und die beiden Grundkörperteile 32, 34 anschließend zusammengedrückt und aneinander befestigt.

Figur 5 zeigt auch Ausbuchtungen 60 im Grundkörper 12 im Innern des Spiegelsaugers. Die Ausbuchtungen 60 bewirken eine Volumenvergrößerung im Innern des Spiegelsaugers 10 und dadurch eine Erhöhung des absaugbaren Luft und/oder Flüssigkeitsvolumens.

Die Figuren zeigen, dass die Verbindungsflächen 36 im Querschnitt des Grundkörpers 10 jeweils einen Winkel von etwa 80 bis 120 Grad, vorzugsweise 90 Grad, mit einer durch die Spiegelfläche 24 des Spiegels 22 ausgebildete Spiegelflächenebene einschließen, wobei die Summe der Winkelbeträge der beiden Grundkörperteile 32, 34 etwa 180 Grad ergibt.

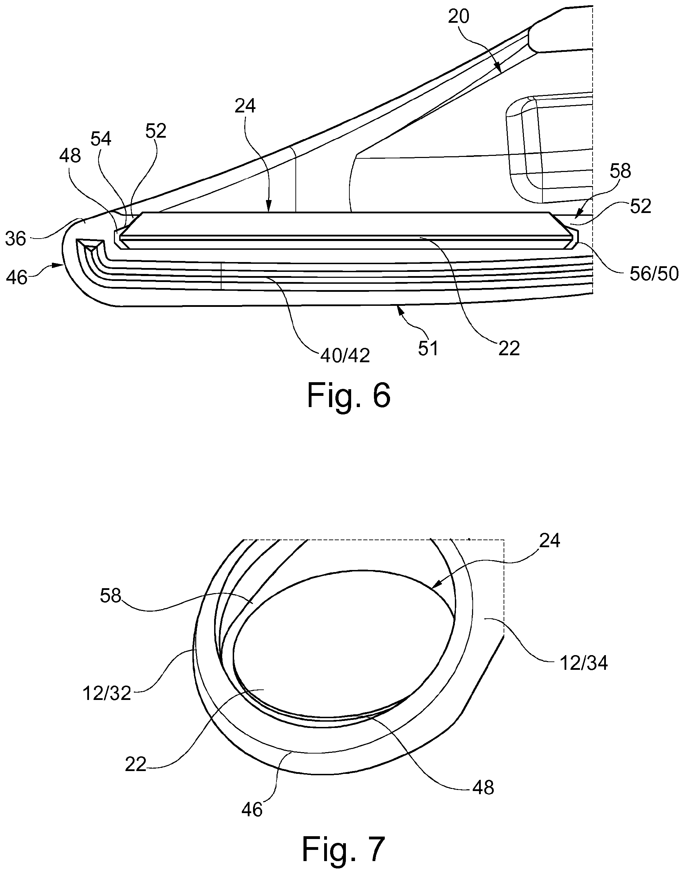

Figur 6 verdeutlicht eine besonders vorteilhafte Ausführungsvariante der Nut/Feder-Verbindung 40. Erkennbar ist, dass die Nut/Feder-Verbindung 40 in Längsrichtung unter den gesamten Spiegel 22 entlang geführt ist. Weiterhin erstreckt sich ein in etwa vertikal verlaufender Abschnitt bis zwischen eine Grundkörpervorderkante 46 und eine Spiegelvorderkante 48.

Bei der Herstellung des Spiegelsaugers 10 verbleibt nach dem Zusammendrücken der beiden Grundkörperteile 32, 34 erfindungsgemäß ein Spalt zwischen den beiden Grundkörperteilen 32, 34, der sich beim anschließenden endgültigen und dauerhaften Verbinden der beiden Grundkörperteile 32, 34 beim Verschweißen mit Kunststoffmaterial oder beim Verkleben mit Klebstoff füllt. Beim bevorzugten Schweißvorgang ist der Spalt derart ausgeführt und dimensioniert, dass er im Wesentlichen exakt die Menge an auftretendem und kaum vermeidbaren weichen oder flüssigen kunststoffmaterial aufnimmt. Hierdurch wird eine durchgehend glatte Außenfläche 16 geschaffen, da ein Materialüberstand nach dem Verkleben verhindert wird.

Je nach Qualitätsanforderung oder Wünschen kann die Außenfläche 16 anschließend geschliffen bzw. geglättet und/oder poliert werden.

Ein weiteres wesentliches Merkmal der Erfindung besteht darin, dass der im gezeigten Ausführungsbeispiel kreisförmige Spiegel 22 in einer Spiegelhalterungsnut 50 gehalten ist. Die Spiegelhalterungsnut 50 umgibt im gezeigten Ausführungsbeispiel den gesamten Außenumfang des Spiegels 22, wobei sich eine Hälfte der Spiegelhalterungsnut 50 im ersten Grundkörperteil 32 und die andere Hälfte im zweiten Grundkörperteil 34 befindet.

Der Spiegel 22 ist im Querschnitt etwa trapezförmig ausgeführt, so dass sein Durchmesser ausgehend von der Spiegelfläche 24 zunimmt, im eingesetzten Zustand also in Richtung der Nut/Feder-Verbindung 40 oder einer Grundkörperunterseite 51. Die Spiegelhalterungsnut 50 weist eine Haltewandung 52 auf, die im eingesetzten Zustand des Spiegels 22 an einer Spiegelaußenseite 54 anliegt. Die Haltewandung 52 umgibt den gesamten Spiegel 22, so dass die Haltewandung 52 einen Freiraum 56 innerhalb der Spiegelhalterungsnut 50 gegenüber einem Innenraum des Grundkörpers 12 abdichtet. Die Abdichtung wird über eine Vorspannung der Haltewandung 52 verbessert. Dies bedeutet, dass der Spiegel 22 beim Einsetzen in die beiden Grundkörperteile 32, 34 gegen die Haltewandung 52 gedrückt wird und diese minimal komprimiert und oder elastisch verformt.

Weiterhin hat dieser Ausführungsvariante den Vorteil, dass der Spiegel 22, wie insbesondere in den Figuren 4, 6 und 7 erkennbar, nahezu bündig in einen Grundkörperboden 58 eingelassen ist.

Die Erfindung ist nicht auf die beschriebenen Ausführungsbeispiele beschränkt, sondern sie erstreckt sich auf alle gleich wirkenden Ausführungsformen. Die beschriebene Ausführungsvariante ist nur beispielhaft und nicht einschränkend zu verstehen. Auch ist es möglich, die gezeigten technischen Merkmale in beliebiger technisch sinnvoller Art und Weise miteinander zu kombinieren.

## Patentansprüche

1. Zahnmedizinischer Spiegelsauger (10) zum Absaugen von Flüssigkeiten und Partikeln aus einem Mundraum eines Patienten, mit einem hohlen Grundkörper (12), der eine Außenfläche (16), eine Innenfläche (14), eine Längsachse (X-X) und eine Ansaugöffnung (20) aufweist, wobei die Innenfläche (14) einen durch die Ansaugöffnung (20) einsehbaren Spiegel (22) aufweist **dadurch gekennzeichnet, dass** der Grundkörper (12) aus einem ersten Grundkörperteil (32) und einem zweiten Grundkörperteil (34) gebildet ist, die über Verbindungsflächen (36) fest miteinander verbunden sind, die beiden Grundköperteile (32, 34) jeweils eine Ausnehmung aufweisen, die im zusammengesetzten Zustand der beiden Grundköperteile (32, 34) eine um den gesamten Außenumfang des Spiegels (22) umlaufende Spiegelhalterungsnut (50) ausbilden, wodurch der Spiegel (22) zwischen den beiden Grundköperteilen (32, 34) unverlierbar gehalten ist.

2. Zahnmedizinischer Spiegelsauger (10) nach Anspruch 1, **dadurch gekennzeichnet, dass** der Durchmesser des Spiegels (22) ausgehend von einer Spiegelfläche (24) in Richtung einer Grundkörperunterseite (51) zunimmt und im zusammengesetzten Zustand der beiden Grundköperteile (32, 34) auch der Durchmesser der Spiegelhalterungsnut (50) in Richtung der Grundkörperunterseite (51) zunimmt und eine Haltewandung (52) der Spiegelhalterungsnut (50) eine Spiegefaußenseite (54) zumindest bereichsweise kontaktiert.

3. Zahnmedizinischer Spiegelsauger (10) nach Anspruch 1 oder Anspruch 2, **dadurch gekennzeichnet, dass** der Spiegel (22) unverklebt ausschließlich durch die Grundkörperteile (32, 34) fest gehalten ist.

4. Zahnmedizinischer Spiegelsauger (10) nach Anspruch 1 oder Anspruch 2, **dadurch gekennzeichnet, dass** der Spiegel (22) mit den Grundkörperteilen (32, 34) verklebt ist.

5. Zahnmedizinischer Spiegelsauger (10) nach einem der Ansprüche 1 bis 4, **dadurch gekennzeichnet, dass** die Grundkörperteile (32, 34) aus einem Kunststoff gefertigt sind.

6. Zahnmedizinischer Spiegelsauger (10) nach einem der Ansprüche 1 bis 5, **dadurch gekennzeichnet, dass** die Verbindungsflächen (36) der beiden Grundköperteile (32, 34) miteinander verschweißt sind.

7. Zahnmedizinischer Spiegelsauger (10) nach einem der Ansprüche 1 bis 6, **dadurch gekennzeichnet, dass** die Verbindungsflächen (36) der beiden Grundköperteile (32, 34) über eine Nut/Feder-Verbindung (40) miteinander verbunden sind.

8. Zahnmedizinischer Spiegelsauger (10) nach Anspruch 7, **dadurch gekennzeichnet, dass** die sich die Nut/Feder-Verbindung (40) in Längsrichtung (X-X) des Grundkörpers (12) zumindest unterhalb des Spiegels (22) und zwischen einer Grundkörpervorderkante (46) und einer Spiegelvorderkante (48) des Spiegels (22) entlang erstreckt.

9. Zahnmedizinischer Spiegelsauger (10) nach einem der Ansprüche 7 bis 8, **dadurch gekennzeichnet, dass** sich die Nut/Feder-Verbindung (40) in Längsrichtung (X-X) im Wesentlichen über die gesamte Länge des Grundköpers (12) erstreckt.

10. Zahnmedizinischer Spiegelsauger (10) nach einem der Ansprüche 1 bis 9, **dadurch gekennzeichnet, dass** die Verbindungsflächen (36) im Wesentlichen in Längsrichtung entlang der Längsachse (X-X) des Grundkörpers (12) verlaufen.

11. Verfahren zur Herstellung eines zahnmedizinischen Spiegelsaugers (10) zum Absaugen von Flüssigkeiten und Partikeln aus einem Mundraum eines Patienten, mit einem hohlen Grundkörper (12), der eine Außenfläche (16), eine Innenfläche (14), eine Längsachse (X-X) und eine Ansaugöffnung (20) aufweist, wobei die Innenfläche (14) einen durch die Ansaugöffnung (20) einsehbaren Spiegel (22) aufweist,
**gekennzeichnet durch** die Verfahrensschritte
- Fertigen eines ersten Grundkörperteils (32) und eines zweiten Grundkörperteils (34), und dabei
- Ausbilden mindestens einer Verbindungsfläche (36) je Grundkörperteil (32, 34),
- Ausbilden einer Spiegelhalterungsnut (50) je Grundkörperteil (32, 34), die derart angeordnet und ausgeformt sind, dass der Spiegel (22) im zusammengesetzten Zustand der beiden Grundköperteile (32, 34) in dieser gehalten ist,
- Einsetzen des Spiegels (22) in die Spiegelhalterungsnut (50) und Zusammenfügen der beiden Grundkörperteile (32, 34) an den Verbindungsflächen (36),
- Befestigen der Grundkörperteile (32, 34) aneinander im Bereich der Verbindungsflächen (36).

12. Verfahren nach Anspruch 11, **gekennzeichnet durch** Ausbilden einer Nut/Feder-Verbindung (40) in den Verbindungsflächen (36) zum Verbinden der Grundkörperteile (32, 34).

13. Verfahren nach Anspruch 11 oder 12, **gekennzeichnet durch** Ausbilden der Nut/Feder-Verbindung (40) in den Verbindungsfläche (36), die sich in etwa in Längsrichtung (X-X) des Grundkörpers (12) zumindest unterhalb des Spiegels (22) und zwischen einer Grundkörpervorderkante (46) und einer Spiegelvorderkante (48) des Spiegels (22) entlang erstreckt.

14. Verfahren nach Anspruch 12 oder Anspruch 13, **dadurch gekennzeichnet, dass** die Nut/Feder-Verbindung (40) derart ausgeführt ist, dass im zusammengefügten Zustand vor dem Befestigen der beiden Grundkörperteile (32, 34) miteinander ein Spalt zwischen den Grundkörperteilen (32, 34) verbleibt.

15. Verfahren nach Anspruch 14, **dadurch gekennzeichnet, dass** die Grundkörperteile (32, 34) im Bereich der Nut/Feder-Verbindung miteinander verschweißt werden, wobei sich der Spalt mit Schweißüberschussmaterial füllt.

16. Verfahren nach Anspruch 14, **dadurch gekennzeichnet, dass** die Grundkörperteile (32, 34) im Bereich der Nut/Feder-Verbindung miteinander verklebt werden, wobei sich der Spalt mit Klebstoff füllt

## Claims

1. A dental mirror suction device (10) for suctioning liquids and particles from an oral cavity of a patient, with a hollow basic body (12) having an outer surface (16), an inner surface (14), a longitudinal axis (X-X) and a suction port (20), wherein the inner surface (14) has a mirror (22) viewable through the suction port (20), **characterized in that** the basic body (12) is formed of a first basic body part (32) and a second basic body part (34) firmly connected via connecting surfaces (36), the two basic body parts (32, 34) each have a recess which, in the assembled state of the two basic body parts (32, 34), form a mirror-retaining groove (50) running around the entire outer circumference of the mirror (22), whereby the mirror (22) is retained in a lossproof manner between the two basic body parts (32, 34).

2. The dental mirror suction device (10) according to claim 1, **characterized in that** the diameter of the mirror (22), starting from a mirror surface (24), increases in the direction of a lower side (51) of the basic body and, in the assembled state of the two basic body parts (32, 34), the diameter of the mirror-retaining groove (50) also increases in the direction of the lower side (51) of the basic body, and a retaining wall (52) of the mirror-retaining groove (50) contacts an outer side (54) of the mirror at least in some portions.

3. The dental mirror suction device (10) according to claim 1 or claim 2, **characterized in that** the mirror (22) is firmly retained, unglued, solely by means of the basic body parts (32, 34).

4. The dental mirror suction device (10) according to claim 1 or claim 2, **characterized in that** the mirror (22) is glued to the basic body parts (32, 34).

5. The dental mirror suction device (10) according to any one of the claims 1 to 4, **characterized in that** the basic body parts (32, 34) are made from a plastic.

6. The dental mirror suction device (10) according to any one of the claims 1 to 5, **characterized in that** connecting surfaces (36) of the two the basic body parts (32, 34) are welded to each other.

7. The dental mirror suction device (10) according to any one of the claims 1 to 6, **characterized in that** connecting surfaces (36) of the two the basic body parts (32, 34) are connected with each other via a tongue-and-groove connection (40).

8. The dental mirror suction device (10) according to claim 7, **characterized in that** the tongue-and-groove connection (40) extends in the longitudinal direction (X-X) of the basic body (12) at least underneath the mirror (22) and between a front edge (46) of the basic body and a mirror front edge (48) of the mirror (22).

9. The dental mirror suction device (10) according to any one of the claims 7 to 8, **characterized in that**, in the longitudinal direction (X-X), the tongue-and-groove connection (40) extends substantially over the entire length of the basic body (12).

10. The dental mirror suction device (10) according to any one of the claims 1 to 9, **characterized in that**, in the connecting surfaces (36) extend substantially in the longitudinal direction along the longitudinal axis (X-X) of the basic body (12).

11. A method for producing a dental mirror suction device (10) for suctioning liquids and particles from an oral cavity of a patient, with a hollow basic body (12) having an outer surface (16), an inner surface (14), a longitudinal axis (X-X) and a suction port (20), wherein the inner surface (14) has a mirror (22) viewable through the suction port (20),
**characterized by** the process steps
- manufacturing a first basic body part (32) and a second basic body part (34), and in the process,
- forming at least one connecting surface (36) per basic body part (32, 34),
- forming one mirror-retaining groove (50) per basic body part (32, 34), which are disposed in formed in such a way that the mirror (22) is retained therein in the assembled state of the two basic body parts (32, 34),
- inserting the mirror (22) into the mirror-retaining groove (50) and joining the two basic body parts (32, 34) at the connecting surfaces (36),
- attaching the basic body parts (32, 34) to each other in the region of the connecting surfaces (36).

12. The method according to claim 11, **characterized by** forming a tongue-and-groove connection (40) in the connecting surfaces (36) for connecting the basic body parts (32, 34).

13. The method according to claim 11 or 12, **characterized by** forming the tongue-and-groove connection (40) in the connecting surfaces (36), approximately extending in the longitudinal direction (X-X) of the basic body (12) at least underneath the mirror (22) and between a front edge (46) of the basic body and a mirror front edge (48) of the mirror (22).

14. The method according to claim 12 or claim 13, **characterized in that** the tongue-and-groove connection (40) is configured in such a way that a gap between the basic body parts (32, 34) remains in the joined state prior to attaching the two basic body parts (32, 34) to each other.

15. The method according to claim 14, **characterized in that** the basic body parts (32, 34) are welded to each other in the region of the tongue-and-groove connection, wherein the gap fills up with excess welding material.

16. The method according to claim 14, **characterized in that** the basic body parts (32, 34) are glued to each other in the region of the tongue-and-groove connection, wherein the gap fills up with adhesive.

## Revendications

1. Aspirateur à miroir (10) dentaire destiné à aspirer, d'une cavité buccale d'un patient, des liquides et particules, comprenant un corps de base (12) creux qui présente une surface extérieure (16), une surface intérieure (14), un axe longitudinal (X-X) et un orifice d'aspiration (20), dans lequel la surface intérieure (14) présente un miroir (22) qui est visible à travers l'orifice d'aspiration (20), **caractérisé par le fait que** le corps de base (12) se compose d'une première partie de corps de base (32) et d'une deuxième partie de corps de base (34) qui sont solidaires l'une de l'autre par des surfaces de liaison (36), que les deux parties de corps de base (32, 34) présentent chacune un évidement qui, à l'état assemblé des deux parties de corps de base (32, 34), forment une rainure de maintien de miroir (50) s'étendant tout autour de l'ensemble de la circonférence extérieure du miroir (22), le miroir (22) étant maintenu ainsi de manière imperdable entre les deux parties de corps de base (32, 34).

2. Aspirateur à miroir (10) dentaire selon la revendication 1, **caractérisé par le fait que** le diamètre du miroir (22) augmente à partir d'une surface de miroir (24) en direction d'une face inférieure de corps de base (51), et que, à l'état assemblé des deux parties de corps de base (32, 34), le diamètre de la rainure de maintien de miroir (50) augmente, lui aussi, en direction de la face inférieure de corps de base (51), et qu'une paroi de maintien (52) de la rainure de maintien de miroir (50) entre en contact, au moins par zones, avec une face extérieure de miroir (54).

3. Aspirateur à miroir (10) dentaire selon la revendication 1 ou la revendication 2, **caractérisé par le fait que** le miroir (22) est retenu, sans être collé, exclusivement par les parties de corps de base (32, 34).

4. Aspirateur à miroir (10) dentaire selon la revendication 1 ou la revendication 2, **caractérisé par le fait que** le miroir (22) est fixé par collage sur les parties de corps de base (32, 34).

5. Aspirateur à miroir (10) dentaire selon l'une quelconque des revendications 1 à 4, **caractérisé par le fait que** les parties de corps de base (32, 34) sont réalisées à partir d'une matière plastique.

6. Aspirateur à miroir (10) dentaire selon l'une quelconque des revendications 1 à 5, **caractérisé par le fait que** les surfaces de liaison (36) des deux parties de corps de base (32, 34) sont soudées entre elles.

7. Aspirateur à miroir (10) dentaire selon l'une quelconque des revendications 1 à 6, **caractérisé par le fait que** les surfaces de liaison (36) des deux parties de corps de base (32, 34) sont reliées entre elles par un joint à rainure et languette (40).

8. Aspirateur à miroir (10) dentaire selon la revendication 7, **caractérisé par le fait que** le joint à rainure et languette (40) s'étend dans la direction longitudinale (X-X) du corps de base (12) au moins au-dessous du miroir (22) et entre un bord avant de corps de base (46) et un bord avant de miroir (48) du miroir (22).

9. Aspirateur à miroir (10) dentaire selon l'une quelconque des revendications 7 à 8, **caractérisé par le fait que** le joint à rainure et languette (40) s'étend dans la direction longitudinale (X-X) pour l'essentiel sur l'ensemble de la longueur du corps de base (12).

10. Aspirateur à miroir (10) dentaire selon l'une quelconque des revendications 1 à 9, **caractérisé par le fait que** les surfaces de liaison (36) s'étendent pour l'essentiel dans la direction longitudinale le long de l'axe longitudinal (X-X) du corps de base (12).

11. Procédé de fabrication d'un aspirateur à miroir (10) dentaire destiné à aspirer, d'une cavité buccale d'un patient, des liquides et particules, comprenant un corps de base (12) creux qui présente une surface extérieure (16), une surface intérieure (14), un axe longitudinal (X-X) et un orifice d'aspiration (20), dans lequel la surface intérieure (14) présente un miroir (22) qui est visible à travers l'orifice d'aspiration (20), **caractérisé par** les étapes de procédé
- fabriquer une première partie de corps de base (32) et une deuxième partie de corps de base (34), et, ce faisant,
- réaliser au moins une surface de liaison (36) par partie de corps de base (32, 34),
- réaliser une rainure de maintien de miroir (50) par partie de corps de base (32, 34) qui sont disposées et formées de telle manière que le miroir (22) est maintenu dans celle-ci lorsque les deux parties de corps de base (32, 34) sont assemblées,
- insérer le miroir (22) dans la rainure de maintien de miroir (50) et assembler les deux parties de corps de base (32, 34) sur les surfaces de liaison (36),
- fixer les parties de corps de base (32, 34) l'une sur l'autre au niveau des surfaces de liaison (36).

12. Procédé selon la revendication 11, **caractérisé par** la réalisation d'un joint à rainure et languette (40) dans les surfaces de liaison (36) pour relier entre elles les parties de corps de base (32, 34).

13. Procédé selon la revendication 11 ou 12, **caractérisé par** la réalisation du joint à rainure et languette (40) dans les surfaces de liaison (36) qui s'étend à peu près dans la direction longitudinale (X-X) du corps de base (12) au moins au-dessous du miroir (22) et entre un bord avant de corps de base (46) et un bord avant de miroir (48) du miroir (22).

14. Procédé selon la revendication 12 ou la revendication 13, **caractérisé par le fait que** le joint à rainure et languette (40) est réalisé de telle manière que, à l'état assemblé, avant de fixer entre elles les deux parties de corps de base (32, 34), une fente reste entre les parties de corps de base (32, 34).

15. Procédé selon la revendication 14, **caractérisé par le fait que** les parties de corps de base (32, 34) sont soudées entre elles au niveau du joint à rainure et languette, ladite fente se remplissant de matériau de soudage en excédent.

16. Procédé selon la revendication 14, **caractérisé par le fait que** les parties de corps de base (32, 34) sont collées entre elles au niveau du joint à rainure et languette, ladite fente se remplissant de colle.
